(19) Europäisches Patentamt
European Patent Office
Office européen des brevets

(11) Publication number : **0 541 497 A1**

# (12) EUROPEAN PATENT APPLICATION

(21) Application number : **92830612.5**

(22) Date of filing : **05.11.92**

(51) Int. Cl.[5] : **C07D 207/16**, C07D 401/12, A61K 31/40

(30) Priority : **06.11.91 IT RM910840**

(43) Date of publication of application : **12.05.93 Bulletin 93/19**

(84) Designated Contracting States : **AT BE CH DE DK ES FR GB GR IE LI LU MC NL PT SE**

(71) Applicant : **Sigma-Tau Industrie Farmaceutiche Riunite S.p.A.**
**Viale Shakespeare, 47**
**I-00144 Rome (IT)**

(72) Inventor : **Giannessi, Fabio**
**Via Abbadia San Salvatore, 16**
**I-00198 Roma (IT)**
Inventor : **Ghirardi, Orlando**
**Via Filippo de Grenet, 69**
**I-00128 Roma (IT)**
Inventor : **Misiti, Domenico**
**Via Bacchiglione, 3**
**I-00199 Roma (IT)**
Inventor : **Tinti, Maria Ornella**
**Via Ernesto Basile, 81**
**I-00182 Roma (IT)**
Inventor : **Cozzolino, Roberto**
**Piazza S. Maria alle Fornaci, 2**
**I-00165 Roma (IT)**

(74) Representative : **Cavattoni, Fabio et al**
**Cavattoni & Raimondi Viale dei Parioli, 160**
**I-00197 Roma (IT)**

(54) **Prolineamide derivatives as enhancers of learning processes and memory and pharmaceutical compositions containing same.**

(57) Prolinamides of formula (1)

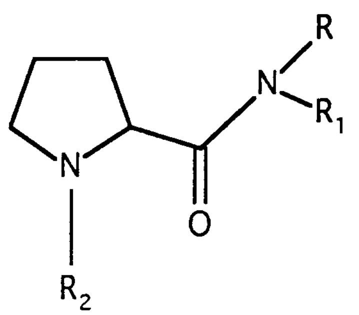

(1)

wherein R is hydrogen, $R_1$ is a straight or branched $C_2$-$C_5$ alkyl group, ω-substituted with a hydroxyl or carboxyl group optionally esterified with a $C_2$-$C_5$ alkanol, or an amino group substituted with one or two straight or branched $C_2$-$C_5$ alkyl groups or an amino acid residue, or a 5- or 6- membered heterocyclic group wherein the heteroatom is sulphur or nitrogen, or R and $R_1$ taken together form a rin of formula

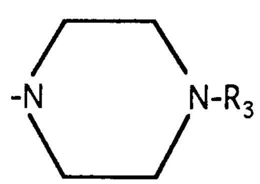

wherein $R_3$ is a phenyl group optionally substituted with halogens or halogen-substituted lower $C_1$-$C_4$ alkyl groups, and $R_2$ is hydrogen, benzyloxycarbonyl or formyl, are potent enhancers of learning and memory processes.

EP 0 541 497 A1

The present invention relates to prolineamides of formula (1)

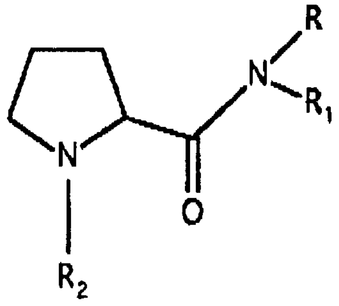

(1)

wherein R is hydrogen, $R_1$ is a straight or branched $C_2$-$C_5$ alkyl group, ω-substituted with a hydroxyl or carboxyl group optionally esterified with a $C_2$-$C_5$ alkanol, or amino group substituted with one or two straight or branched $C_2$-$C_5$ alkyl groups or amino acid residue, or a 5- or 6-membered heterocyclic group wherein the heteroatom is sulphur or nitrogen, or R and $R_1$ taken together form a ring of formula

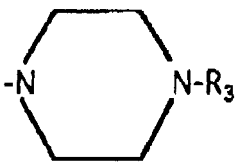

wherein $R_3$ is a phenyl group optionally substituted with halogens (particularly fluorine and chlorine) or halogen-substituted lower $C_1$-$C_4$ alkyl groups, and $R_2$ is hydrogen, benzyloxycarbonyl or formyl.

In particular, $R_1$ is preferably selected from
2- hydroxyethyl
3- carboxypropyl
2- (N,N-diisopropyl) aminoethyl,
2- (N-benzyloxycarbonylprolyl) aminoethyl; and
3- pyridyl.
$R_3$ is preferably selected from 3- trifluoromethylphenyl and 3-chlorophenyl.

The compounds of formula (1) are nootropic substances potent enhancers of learning processes and memory.

The present invention also relates to orally or parenterally administrable pharmaceutical compositions for enhancing the learning processes and memory, comprising a compound of formula (1) as active ingredient.

As for the prior art, L-pyroglutamic acid (5-oxo-L-proline) which, from a structural viewpoint, is vaguely related to the compounds of the present invention, is known to exhibit nootropic activity, however, at doses 5-10 times as high as those of oxiracetan (Pharm. Re. Comm. 19, 901-912, 1987).

Other known nootropic compounds, structurally less vaguely related to the present compounds, however still considerably distinct therefrom, are 1-(carbobenzoxy-L-prolyl)-2-pyrrolidone disclosed in the European patent application EP 0412058 and a class of 1-alkanoyl or 1-phenylalkanoylprolinamides disclosed in Khim. Farm. Zh. 23/3, 276, 1989. (C.A. 111, 166767 r.).

Among the compounds available on the market, the closest ones to the compounds of formula (1) are Piracetam (Curr. Rev. Psycopharm 3, 22, 1976) and Oxiracetam (Il Farmaco ed. Sci. 39/1, 16, 1984.

As will be hereinbelow shown, the compounds of the present invention are more potent than the known compounds.

Since proline nucleus contains one chiral carbon atom the compounds of formula (1) can exist as two enantiomers designated (R) and (S); since the $R_1$ group can contribute a further chiral center, the compounds of formula (1) can also exist as diastereomers; in both cases, the compounds of formula (1) can also exist as racemic mistures. Since it has been found that both the optically active forms and the racemic mixtures are pharmacologically active, hereinbelow, for the sake of semplicity, no specific reference to the optical activity of the compounds shall be made.

The compounds of formula (1) wherein $R_2$ is benzyloxycarbonyl are prepared via the process shown in the following synthesis scheme I:

Scheme I

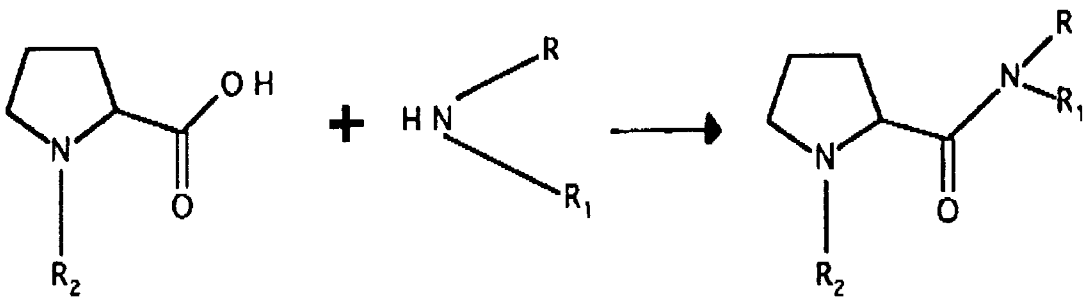

N-benzyloxycarbonylproline is activated with a condensing agent such as carbonyldiimidazole (CDI), dicyclohexylcarbodiimide (DCC), 2-ethoxy-1-ethoxycarbonyl-1,2-dihydroquinoline (EEDQ), or it is activated as short-chain alkyl ester in an inert, anhydrous organic solvent, such as acetonitrile or methylene chloride, for reaction times between 1 and 24 hours, at a temperature of 20°C-80°C. A stoichiometric or excess amount of the selected amine is then added and the resulting solution is kept under stirring for time periods comprised between 20 hours and 60 hours at 20°C-80°C. The raw reaction product is purified by silica gel chromatography using as eluant ethyl acetate or ethyl acetate under hexane or methanol gradient.

When $R_2$ is hydrogen, the product of formula (1) wherein $R_2$ is benzyloxycarbonyl is subjected to hydrogenolysis in a hydroalcoholic phase in the presence of a Pd/C catalyst, as shown in the following reaction scheme II:

Scheme II

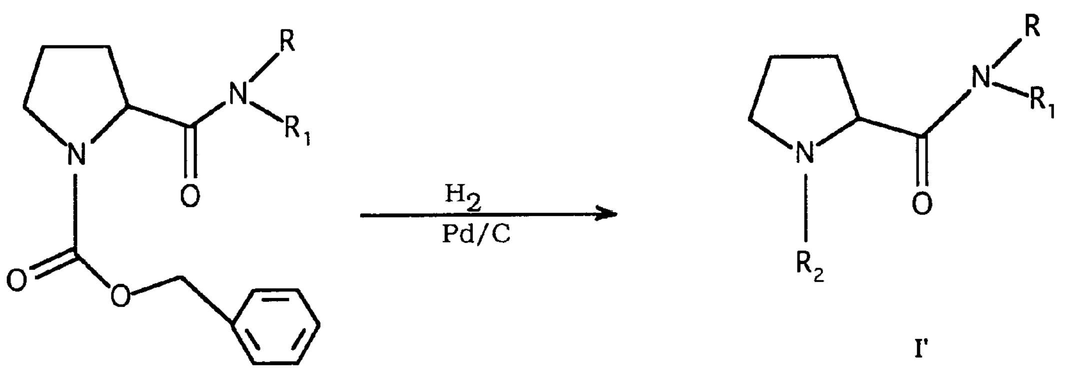


I'

When $R_2$ is formyl, the reaction is as shown in the following synthesis scheme III:

Scheme III

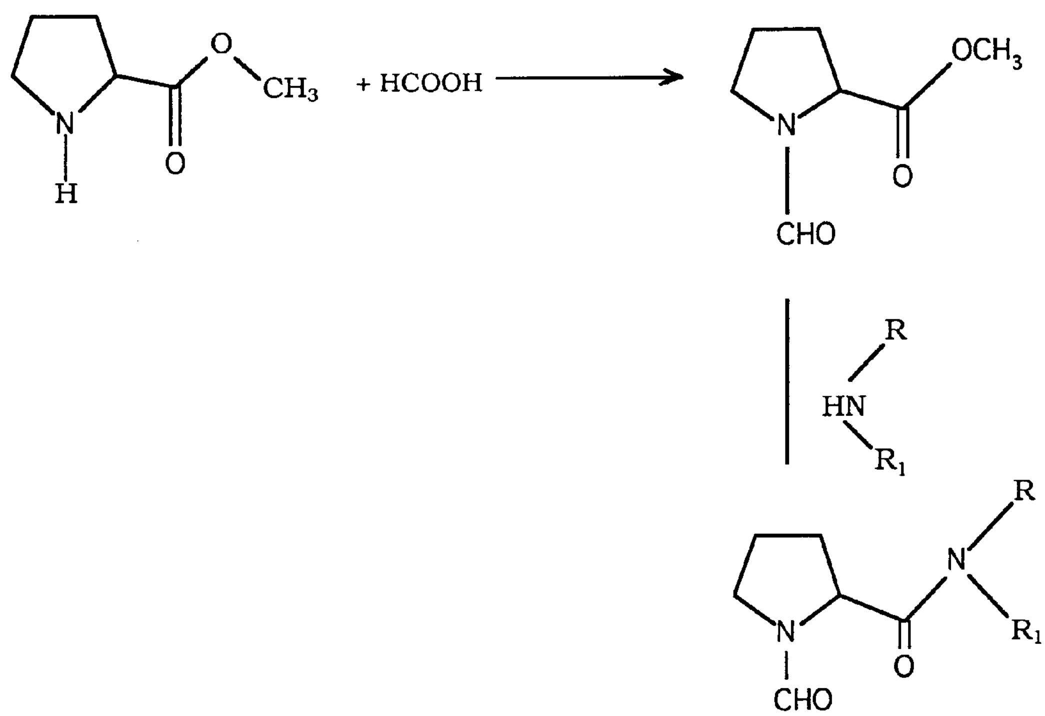


I''

**EXAMPLE 1**

PREPARATION OF (S)-N-[N-(BENZYLOXYCARBONYL)-PROLYL]-3-AMINOPYRIDINE (ST 790).

DCC (2.98 g, 14.43 mmoles) was added to (S)-N-benzyloxycarbonyl)-proline (3 g, 12.03 mmoles) in $CH_3CN$ (100 ml) and the mixture was refluxed for one hour. 3-aminopyridine (1.13 g, 12.03 mmoles) was added and the mixture was refluxed for two days, cooled and filtered. The filtrate was evaporated to dryness and the residue was chromatographed on silica gel using EtOAc as eluant. 2.26 g of product were obtained.

Yield = 57 %

$[\alpha]_D^{25}$ = - 69.5° MeOH (c=1)

TLC = silica gel

Eluant = EtOAc

RF= 0.3

| Elementary analysis for $C_{18}H_{19}N_3O_3$ | | | |
|---|---|---|---|
| Calculated | C 66.47 | H 5.88 | N 12.91 |
| Found | C 65.8 | H 5.95 | N 12.77 |

$^1H$ NMR ($CDCl_3$): δ 9.2(br,1H,-CONH-),8.5-7.7 (m,3H,pyridine), 7.2-6.9(m,6H,benzyl, pyridine), 5.1(s,2H, $PhCH_2O$-),4.4(m,1H,-CHCON-), 3.65-3.35(m,2H,-$CH_2$NCO-), 2.4-1.7(m,4H,-$CH_2CH_2$CH-)

HPLC

μ Bondapack -$C_{18}$ Length = 300 mm, inner diameter = 3.9 mm,

size = 10 μm

Eluant = $KH_2PO_4$ 0.05 M/$CH_3CN$ (65:35)

Flow rate = 1 ml/min

Permanence time = 5.53 min

**EXAMPLE 2**

PREPARATION OF (S)-1-[N-(BENZYLOXYCARBONYL)-PROLYL]-4-(3-TRIFLUOROMETHYLPHENYL)-PIPERAZINE (ST 791)

EEDQ (3.155 g, 12.75 mmoles) was added to (S)-N-(benzyloxycarbonyl)-proline (3 g, 12.03 mmoles) and 1-(3-trifluoromethylphenyl) piperazine (2.77 g, 12.03 mmoles) in $CH_3CN$ (150 ml) and the solution was kept under stirring for 20 hours at room temperature. The solvent was evaporated and the oily residue was chromatographed on silica gel using EtOAc-hexane 8:2 as eluant. 3 g of product were obtained.
Yield = 54 %
$[\alpha]_D^{25}$ = - 8.2° MeOH (c = 1.3)
TLC = silica gel
Eluant = EtOAc
RF = 0. 73

| Elementary analysis for $C_{24}H_{26}F_3N_3O_3$ | | | |
|---|---|---|---|
| Calculated | C 62.46 | H 5.68 | N 9.10 |
| Found | C 62.48 | H 6.06 | N 9.77 |

$^1H$ NMR ($CDCl_3$): δ 7.4-6.95(m,9H,aromatic),5.25-4.98 (m,2H,Ph$\underline{CH_2}$O-) 4.8-4.62(m,1H,-$\underline{CH}$CON-),3.9-2.7(m,10H,-$\underline{CH_2}$NCO-,piperazine), 2.28-1.8(m,4H,-$\underline{CH_2}\underline{CH_2}$CH-)
HPLC
μ Bondapack -$C_{18}$ Length = 300 mm, inner diameter = 3.9 mm,
size = 10 μm
Eluant = $KH_2PO_4$ 0.05 M/$CH_3CN$ (50:50)
Flow rate = 1 ml/min
Permanence time = 15.52 min.

**EXAMPLE 3**

PREPARATION OF (S)-1-[N-(BENZYLOXYCARBONYL)-PROLYL]-4-(3-CHLOROPHENYL)-PIPERAZINE (ST 792)

Prepared as in Example 1 from 1-(3-chlorophenyl)-piperazine. Chromatography was carried out using EtOAc-Hexane 8:2 as eluant
Yield = 50%
$[\alpha]_D^{25}$ = - 8.4° MeOH (c = 1)
TLC = silica gel
Eluant = EtOAc
RF = 0.67

| Elementary analysis for $C_{23}H_{26}ClN_3O_3$ | | | |
|---|---|---|---|
| Calculated | C 64.55 | H 6.12 | N 9.82 |
| Found | C 64.30 | H 6.32 | N 9.60 |

$^1H$ NMR($CDCl_3$): δ 7.4-6.68(m,9H,aromatic), 5.25-5 (m,2H,Ph$\underline{CH_2}$,O-), 4.8-4.62 (m,1H,-$\underline{CH}$CON-),3.9-2.7 (m,10H,$\underline{CH_2}$,NCO-,piperazine), 2.28-1.85 (m,4H,-$\underline{CH_2}\underline{CH_2}$CH-)
HPLC
μ Bondapack -$C_{18}$ Lenght = 300 mm, inner diameter = 3.9 mm,
size = 10 μm
Eluant = $KH_2PO_4$ 0.05 M/$CH_3CN$ (50:50)
Flow rate = 1 ml/min

Permanence time = 13.08 min

## EXAMPLE 4

PREPARATION OF (S)-N-[N-(BENZYLOXYCARBONYL)-PROLYL]-ETHANOLAMINE (ST 704).

EEDQ (7.44 g, 30.08 mmoles) was added to (S)-N-benzyloxycarbonyl)-proline (5 g, 20.06 mmoles) in $CH_3CN$ (250 ml) and the solution was kept under stirring for one hour at room temperature, Ethanolamine was added (1.84 g, 30.08 mmoles) and the resulting solution kept at room temperature for 60 hours. $Et_2O$ was added to the residue following evaporation of the solvent and the solid thus formed was filtered off, dissolved in a little amount of MeOH and precipitated again with $Et_2O$, 4.98 g of product were obtained.
Yield = 85 %
Melting point = 103-104° C
$[\alpha]_D^{25}$ = 61° $H_2O$ (c=1)
TLC = silica gel
Eluant = EtOAc
RF = 0.4

| Elementary analysis for $C_{15}H_{20}N_2O_4$ | | | |
|---|---|---|---|
| Calculated | C 61.63 | H 6.89 | N 9.58 |
| Found | C 61.88 | H 7.15 | N 9.77 |

$^1$HNMR($CDCl_3$):δ7.18(s,5H,aromatic),6.8(br,1H,CONH), 5(m,2H,Ph$CH_2$CO-)4.3(m,1H,-CHCON-), 3.7-3.0(m, 7H,-$CH_2$NCO-,-N$CH_2CH_2$OH), 2.35-1.7 (m,4H,$CH_2CH_2$CH)
HPLC
Lichrosorb -$RP_8$ Length = 250 mm, inner diameter = 4 mm,
size = 5 μm
Eluant = $CH_3CN/KH_2PO_4$ 0.05 M (75:25)
Flow rate = 1 ml/min
Permanence time = 8.83 min

## EXAMPLE 5

PREPARATION OF (S)-N-[N-(BENZYLOXYCARBONYL)-PROLYL]-4-AMINOBUTYRIC ACID (ST 823).

CDI (2.43 g, 15 mmoles) was added to (S)-N-benzyloxycarbonyl)-proline (3.4 g, 13.64 mmoles) in $CH_2Cl_2$ (50 ml) and the solution was kept under stirring for one hour at room temperature. 4-aminomethylbutyrate hydrochloride (2.095 g, 13.64 mmoles) was added and the resulting solution was kept under stirring for 20 hours at room temperature. The solution was washed with 1N HCl, 1N NaOH, $H_2O$ and a saturated solution of NaCl and dried on anhydrous $Na_2SO_4$ . 2.1 g (6.02 mmoles) of (S)-N-(N-benzyloxycarbonyl)-propyl-4-aminomethylbutyrate (TLC = silca gel Eluant=EtOAc-MeOH 9:1 RF = 0.71) were obtained, which were then dissolved in 2H HCl (30 ml) and kept under stirring for 24 hours at room temperature. The aqueous solution was extracted with $CH_2Cl_2$ (3 X 30 ml) and the combined organic phases were dried on anhydrous $Na_2SO_4$ . Following solvent evaporation, 1.6 g of product were obtained that were further purified by chromatography on silica gel using EtOAc-MeOH 9 :1 as eluant. 0.85 g of product were obtained.
Overall yield = 18.6 %
Melting point = 77-79 ° C
$[\alpha]_D^{25}$ = - 42.9 MeOH (c=1)
TLC = silica gel
Eluant = EtOAc-MeOH 9:1
RF = 0.18

| Elementary analysis for $C_{17}H_{22}N_2O_5$ | | | |
|---|---|---|---|
| Calculated | C 61.06 | H 6.63 | N 8.3 |
| Found | C 60.52 | H 6.86 | N 8.7 |

$^1$HNMR($CDCl_3$):δ10.5(s,1H,COOH),7.38(s,5H,aromatic), 7(br,1H,-CONH), 5.2-5 (m,2H,-Ph$CH_2$O-), 4.38 (m, 1H,-CHCON-) 3.6-3.05 (m,4H,-$CH_2$NCO-$CH_2$NHCO), 2.4-1.6 (m,8H,-$CH_2CH_2$COOH,$CH_2CH_2$CH-)
HPLC
μ Bondapack -$C_{18}$ Length = 300 mm, inner diameter = 3.9 mm,
size = 10 μm
Eluant = $KH_2PO_4$ 0.05 M/$CH_3CN$ (60:40)
Flow rate = 1 ml/min
Permanence time = 4.24 min

## EXAMPLE 6

PREPARATION OF (S,S)-1.2-BIS-[N-(BENZYLOXYCARBONYL)-PROLYL]-ETHYLENDIAMINE (ST 695).

DCC (2.27 g, 11 mmoles), and ethylendiamine (0.3 g, 5 mmoles) were added to (S)-N-(benzyloxycarbonyl)-proline (2.5 g, 10 mmoles) in $CH_2Cl_2$ (50 ml) and the mixture was refluxed for two days. The mixture was cooled and filtered, the filtrate evaporated to dryness and the residue chromatographed on silica gel using EtOAc-MeOH 9 :1 as eluant, 1.3 g of product were obtained.
Yield = 50 %
Melting point = 158-159 °C
$[\alpha]_D^{25}$ = - 22.5° $CHCl_3$ (c=5)
TLC = silica gel
Eluant = EtOAc-MeOH 9:1
RF= 0.67

| Elementary analysis for $C_{28}H_{34}N_4O_6$ | | | |
|---|---|---|---|
| Calculated | C 64.35 | H 6.35 | N 10.72 |
| Found | C 64.32 | H 6.60 | N 10.35 |

$^1$H NMR ($CDCl_3$): δ 7.2(s,10H,aromatic), 7(br,2H,2-CONH-), 5(s,4H,2Ph$CH_2$O-), 4.15 (m,2H,2-CHCON-), 3.65-2.9(m,8H,2-$CH_2$NCO-,-N$CH_2CH_2$N-), 2.35-1.5(m,8H,2-$CH_2CH_2$CH-)
HPLC
μ Bondapack -$C_{18}$ Length = 300 mm, inner diameter = 3.9 mm,
size = 10 μm
Eluant = $CH_3CN/KH_2PO_4$ 0.05 M (60:40)
Flow rate = 1 ml/min
Permanence time = 4.83 min

## EXAMPLE 7

PREPARATION OF (S)-1-[N-(BENZYLOXYCARBONYL)-PROLYL]-2,2(DIISOPROPYL)-ETHYLENDIAMINE (ST 691).

DCC (10.34 g, 50 mmoles), and N,N-diisopropylethylendiamine (7.23 g, 50 mmoles) were added to (S)-N-(benzyloxycarbonyl)-proline (12,5 g, 50 mmoles) in $CH_2Cl_2$ (300 ml) and the mixture was refluxed for two days. The mixture was cooled and filtered, the filtrate evaporated to dryness and the residue chromatographed on silica gel using EtOAc-MeOH 95:5 as eluant, 5 g of an oily product were obtained.
Yield = 27 %
$[\alpha]_D^{25}$ = - 49.97° MeOH (c=5)
TLC = silica gel
Eluant = EtOAc-MeOH 9:1

RF = 0.3

| Elementary analysis for $C_{21}H_{33}N_3O_3$ | | | |
|---|---|---|---|
| Calculated | C 67.17 | H 8.66 | N 11.19 |
| Found | C 66.5 | H 9.07 | N 11.04 |

$^1$H NMR ($CDCl_3$): δ 7.2-(s,5H,aromatic), 6.8(br,1H,$CO\underline{NH}$-), 5 (s,2H,$Ph\underline{CH_2}O$-), 4.3 (m,1H,-$\underline{CH}CON$-), 3.65-2.35(m,8H,-$\underline{CH_2}NCO$-,-$CONH\underline{CH_2CH_2}N$,$2(CH_3)_2\underline{CH}$-), 2.35-1.6(m,4H,-$\underline{CH_2CH_2}CH$-), 1(d,12H,$2(CH_3)_2CH$-)
HPLC
μ Bondapack -$C_{18}$ Length = 300 mm, inner diameter = 3.9 mm,
size = 10 μm
Eluant = $CH_3CN/KH_2PO_4$ 0.05 M (35:65)
Flow rate = 2 ml/min
Permanence time = 2.93 min

**EXAMPLE 8**

PREPARATION OF (S)-1-[N-(FORMYL)-PROLYL]-2.2(DIISOPROPYL)-ETHYLENDIAMINE (ST 849).

CDI ( 12.30 g. 76 mmoles) was added to formic acid (3. 175 g. 69 mmoles) in $CH_2Cl_2$ (200 ml) and the solution was kept under stirring for one hour at room temperature. (S)-proline methylester hydrochloride (11.42 g, 69 mmoles) was added and the solution kept under stirring for 20 hours at room temperature. The solution was washed with 1N HCl. 1N NaOH, $H_2O$ and a saturated solution of NaCl and dried on anhydrous $Na_2SO_4$. 5 g (32 mmoles) of (S)-N-(formyl)-proline methyl ester (TLC = silica gel Eluant=EtOAc-MeOH 8:2 RF = 0.69) were obtained to which N,N-diisopropylethylendiamine (4.6 g. 32 mmoles) was added. The mixture of the two oily products was kept under stirring for 20 hours at room temperature. Following removal of the methanol that formed by evaporation under vacuum, the oil was chromatographed on silica gel using EtOAc-MeOH- (32 %) $NH_4OH$ (16:4:0.3) as eluant yielding 2.26 g of hydroscopic product that was taken up with $H_2O$ and lyophilized to yield 2.03 g of oily product.
Overall yield = 11 %
$[\alpha]_D^{25}$ = - 49.5° $CHCl_3$ (c=1)
TLC = silica gel
Eluant = EtOAc-MeOH 8:2
RF = 0.1

| Elementary analysis for $C_{14}H_{27}N_3O_2$ | | | |
|---|---|---|---|
| Calculated | C 62.24 | H 10.10 | N 15.59 |
| Found | C 61.3 | H 10.07 | N 15.02 |

$^1$HNMR($CDCl_3$):δ8.3(2s,1H,-$CO\underline{H}$),7.2 and 6.9(2br,1H,-$CO\underline{NH}$), 4.5-4.35 (m,1H,-$\underline{CH}CON$-)3.7-3.42 (m,2H, -$\underline{CH_2}NCO$-) 3.35-3.15(m,2H,-$\underline{CH_2}NHCO$-), 3.1-2.95(m,2H,$2(CH_3)_2CH$-), 2.66-2.55(m,2H,-$CONHCH_2\underline{CH_2}N$-), 2.45-1.8(m,4H,-$\underline{CH_2CH_2}CH$-), 1.05(d,12H,$2(CH_3)_2CH$-)
HPLC
μ Bondapack -$C_{18}$ Length = 300 mm, inner diameter = 3.9 mm,
size = 10 μm
Eluant = $KH_2PO_4$ 0.05 M/$CH_3CN$ (85:15)
Flow rate = 1 ml/min
Permanence time = 5.41 min

Assessement of the antiamnesic activity

In order to assess the antiamnesic activity the passive avoidance test in mince was used. Amnesia was brought about by electroconvulsive shock (ECS) (cfr. Banfi et al., A screening method for substances potentially active on learning and memory. J. Pharmacol. Methods Vol.: 8 (4) 225-263, 1982).

Male CDI mice weighing 25-26 g (charles River, Germany) fed on a normal diet, were used.

The compounds were administered i.p.; doses equimolar to 9.0 and 0.9 mg piracetam $kg^{-1}$ were used.

The water-soluble compounds were dissolved in saline; the insoluble ones were dissolved in dimethylsulfoxide and then diluted in 2% Tween 80, ratio 1:4.

The apparatus for passive avoidance conditioning was a black plastic chamber (42 x 42 cm, height 40 cm), provided with a floor constructed of metal that could be electrified. From the front wall extended a white runway, 30 cm long and 10 cm wide provided with side walls 12 cm high, which led into the box through a guillotine door. The runways was lightened by a 60 W lamp (cfr. Ader et al., Retention of a passive avoidance response as a function of the intensity and duration of electric shock. Psychon. Scj., 26 (3), 125-127, 1972).

Experimental method

The animals, 30 minutes following treatment, were placed on the runways. After one minute of adaptation, the door was raised and the time employed by the animal to enter the darkened with all four feet, was recorded.

Upon entry, the guillotine door was lowered and 3 seconds thereafter the rods were electrified, 0.24 mA for 2 seconds.

The mouse was then removed from the chamber and immediately administered and electroshock delivered through spring clips attached to the ears (square wave, intensity 20 mA, amplitude 0.6 msec, duration 0.5 s, frequency 50 Hz). Immediately thereafter the animal was placed in the housing cage. Retention was assessed 24 hours later by placing the animal on the runways and again evaluating the latency in entering the chamber, using an end-point of 300 s (crf. Brammer, loc. cit.).

In each experiment, two groups of animals in addition to the treated ones were used, that were defined as follows:

(1) ceiling control animals (treated with placebo and not subjected to ECS amnesia treatment) to ensure that these animals not treated with the amnesia agent remembered the task;

(2) base-line control animals (treated with placebo and subjected to ECS amnesia treatment) to ensure that ECS produced amnesia in the animals not treated with the compounds of the present invention.

The results of each compound under examination were expressed as percentage of amnesia reversal (AR) in order to make comparisons across the tested compounds.

AR is defined as follows: $AR = \frac{CIt}{CIc} \cdot 100$

wherein CI, Comparison Index (the subscripts "t" and "c" refer to "treated" and "ceiling control", respectively) is defined by the formula

$$CI = [\Sigma Aij/Ni.Nj)]100$$

wherein

- Ni is the number of animals belonging to the i-nth group (ceiling control or treated animals);
- Nj is the number of animals belonging to the j-nth group (base-line control animals); and
- Aij is a binary function that can take only the values + 1,0 or -1 depending on whether the latency time (in seconds) of an animal belonging to the i-nth group, Xi, is higher than, the same as or smaller than the latency time (in seconds) of an animal of the j-nth group, Xj.

The sum ΣAij encompasses all the possible pairs obtained by combining each term Xi with each term Xj.

Whenever in performing the test the Comparison Index (CI) between ceiling control animals and base-line control animals, generally expected to range between 60 and 80%, turned out to be lower than 40% the data for the whole experiment were discarded.

The results are shown in Table 1.

Table 1

Passive avoidance following ECS-induced amnesia

The table shows the ARs of some compounds of the present invention. The number of animals (No.) and the AR of each compound tested at various dose levels are reported.

ECS

| | 9mg/kg | | 0.9 mg/kg | |
|---|---|---|---|---|
| | n° | %AR | n° | %AR |
| Ceiling control group | 62 | 100 | 53 | 100 |
| Base-line control group | 102 | 0 | 87 | 0 |
| Piracetam | 30 | 0 | 27 | 0 |
| ST 691 | 12 | 0 | 24 | 52 |
| ST 704 | 23 | 46 | 11 | 0 |
| ST 792 | 12 | 0 | 12 | 26 |
| ST 849 | 24 | 59 | 12 | 3 |

Behavioural profile

The behavioural profile was assessed in male CDI Mice (Charles River, Italy) weighing 22-24 g, using the Irwin test (IRWIN S., Drug screening and evaluation procedures; 136, 123-128, 1962). The animals had been caged under normal conditions and kept fasting for the last 18 hours.

Following administration of the compounds, the behaviour of the animals was monitored for 6 hours. The compounds were suspended in 10% arabic gum and orally administered at doses equimolar to 90, 23, 5.4 and 1.4 mg piracetam/10mL/kg of body weight.

The animals of the control groups were administered 10% arabic gum (10mL/kg, orally).

No compound altered, at the tested doses, the behavioural profile except ST 691 which showed to be toxic (death rate: 100%) at 90 mg/kg, but non toxic at the other doses.

Analgesic activity

The analgesic activity was assessed in CDI Mice (Charles River, Italy) weighing 22-24 g, utilizing the hot plate test (56° C).

The animals, kept under normal caging conditions and kept fasting for 18 hours, were placed on the hot

plate for 30, 60, 120 and 180 minutes following the oral administration of 90, 23, 5.4 and 1.4 mg/10 mL/kg equimolar to piracetam of each compound under examination.

The analgesic activity was assessed by measuring the increase (in seconds) of the time the animals continued to stay on the hot plate. None of the tested compounds was shown to possess analgesic activity.

The compounds of the present invention can be formulated into orally or parenterally administrable pharmaceutical compositions. Suitable excipient and compositions for tablets, vials and like are illustrated in the Canadian patent 1.100.515.

Pharmaceutical compositions in unit dosage form comprise between about 100 and about 500 mg of active ingredient.

## Claims

1. Prolineamides of formula (1)

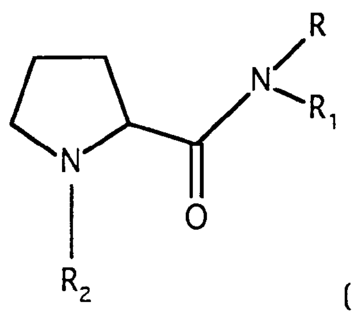

(1)

wherein R is hydrogen, $R_1$ is a straight or branched $C_2$-$C_5$ alkyl group, ω-substituted with a hydroxyl or carboxyl group optionally esterified with a $C_2$-$C_5$ alkanol, or an amino group substituted with one or two straight or branched $C_2$-$C_5$ alkyl groups or an amino acid residue, or a 5- or 6-membered heterocyclic group wherein the heteroatom is sulphur or nitrogen, or R and $R_1$ taken together form a ring of formula

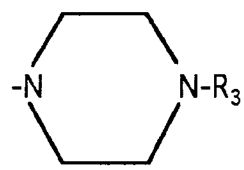

wherein $R_3$ is a phenyl group optionally substituted with halogens or halogen-substituted lower $C_1$-$C_4$ alkyl groups, and $R_2$ is hydrogen, benzyloxycarbonyl or formyl.

2. Prolinamide according to claim 1, wherein $R_1$ is selected from 2-hydroxyethyl, 3-carboxypropyl, 2-(N,N-diisopropyl)-aminoethyl, 2-(N-benzyloxycarbonylprolyl)-aminoethyl and 3-pyridyl.

3. Prolineamide according to claim 1 wherein $R_3$ is selected from 3-trifluoromethylphenyl and 3-chlorophenyl.

4. (S)-N-[N-(benzyloxycarbonyl)-prolyl]-3 aminopyridine.

5. (S)-1-[N-(benzyloxycarbonyl)-prolyl]-4-(3-trifluoromethylphenyl)-piperazine.

6. (S)-1-[N-(benzyloxycarbonyl)-prolyl]-4-(3-chlorophenyl)-piperazine.

7. (S)-N-[N-(benzyloxycarbonyl)-prolyl]-ethanolamine.

8. (S)-N-[N-(benzyloxycarbonyl)-prolyl]-4-amino-butyric acid.

9. (S,S)-1,2-bis-[N-(benzyloxycarbonyl)-prolyl]-ethylenediamine.

10. (S)-1-[N-(benzyloxycarbonyl)-prolyl]-2,2-(diisopropyl)-ethylene diamine.

11. (S)-1-[N-(formyl)-prolyl]-2,2-(diisopropyl)-ethylendiamine.

12. An orally or parenterally administrable pharmaceutical composition for enhancing the processes of learning and memory comprising as active ingredient a prolineamide of formula (1)

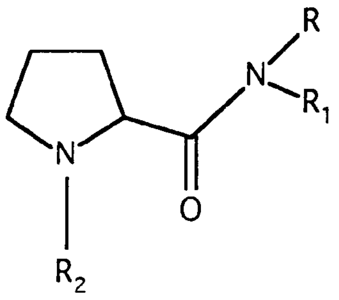

(1)

wherein R is hydrogen, $R_1$ is a straight or branched $C_2$-$C_5$ alkyl group, ω-substituted with a hydroxyl or carboxyl group optionally esterified with a $C_2$-$C_5$ alkanol, or an amino group substituted with one or two straight or branched $C_2$-$C_5$ alkyl groups or an amino acid residue, or a 5- or 6- membered heterocyclic group wherein the heteroatom is sulphur or nitrogen, or R and $R_1$ taken together form a ring of formula

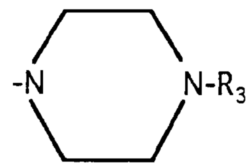

wherein $R_3$ is a phenyl group optionally substituted with halogens or halogen-substituted lower $C_1$-$C_4$ alkyl groups, and $R_2$ is hydrogen, benzyloxycarbonyl or formyl.

13. The pharmaceutical composition of claim 12 in unit dosage form, comprising from about 100 to about 500 mg of a prolineaminde of formula (1).

European Patent Office

# PARTIAL EUROPEAN SEARCH REPORT

which under Rule 45 of the European Patent Convention shall be considered, for the purposes of subsequent proceedings, as the European search report

Application Number
EP 92 83 0612
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | EP-A-0 268 281 (SUNTORY)<br>25 May 1988<br>* the whole document *<br>--- | 1-13 | C07D207/16<br>C07D401/12<br>A61K31/40 |
| A | EP-A-0 123 977 (CASSELLA)<br>7 November 1984<br>* example 4 *<br>--- | 1-13 | |
| X | EP-A-0 221 019 (NIPPON ZOKI PHARMACEUTICAL CO. LTD.)<br>6 May 1987<br>* RN 112558-77-7 : Z-Pro-GABA<br>* page 11; example 4 *<br>* Compound 18: Pro-EACA<br>* page 13 *<br>--- | 1,2,8,<br>12,13 | |
| X | US-A-4 511 390 (J. M. KAUER)<br>16 April 1985<br>* claims 1-6 *<br>* examples 1-10 *<br>---<br>-/-- | 1,2,7 | |
| | | | TECHNICAL FIELDS SEARCHED (Int. Cl. 5) |
| | | | C07D |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 01 MARCH 1993 | Bernd Kissler |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number
EP 92 83 0612
Page 2

| Category | Citation of document with indication, where appropriate, of relevant passages | Relevant to claim | CLASSIFICATION OF THE APPLICATION (Int. Cl. 5) |
|---|---|---|---|
| X | EP-A-0 123 444 (YAMANOUCHI PHARMACEUTICAL CO. LTD.)<br>31 October 1984<br>* compounds 53 and 79 on pages 73-74 *<br>--- | 1,2,7, 12,13 | |
| A | CHEMICAL ABSTRACTS, vol. 53<br>Columbus, Ohio, US;<br>abstract no. 4260b,<br>* abstract *<br>& CHEM. BER.<br>vol. 91, 1958,<br>pages 2066 - 2073<br>H. MIX, F. W. WILCKE, W. LANGENBECK 'Organic Catalysts'<br>--- | 1 | |
| | | | TECHNICAL FIELDS SEARCHED (Int. Cl. 5) |
| A | CHEMICAL ABSTRACTS, vol. 109,<br>1988, Columbus, Ohio, US;<br>abstract no. 170371,<br>* abstract *<br>& INDIAN J. CHEM., SECT. B<br>vol. 26B, no. 8, 1987,<br>pages 748 - 751<br>SHARMA, S. ET. AL. 'Studies in potential filaricides'<br>--- | 1 | |
| X | CHEMICAL AND PHARMACEUTICAL BULLETIN.<br>vol. 35, no. 3, 1987, TOKYO JP<br>pages 1249 - 1254<br>KAZUHARU I. ET. AL. 'syntheses and properties of dipeptides containing gamma-aminobutyric acid or its analogues at the c-terminal.'<br>* Example 31: Z-Pro-GABA-OH on page 1254 *<br>* Example 6: H-Pro-GABA-OH on page 1254 *<br>* Example 37: Z-Pro-EACA-OH on page 1254 *<br>--- | 1,2,8 | |
| A | CHEMICAL ABSTRACTS, vol. 73,<br>1970, Columbus, Ohio, US;<br>abstract no. 181,<br>* RN 17268-28-9 (Prolinamide, N-(N'-dimethylaminoethyl)-<br>* abstract *<br>& BIOCHEM. J.<br>vol. 117, no. 2, 1970,<br>pages 247 - 256<br>---<br>-/-- | 1 | |

EPO FORM 1503 03.82 (P04E10)

European Patent Office

# PARTIAL EUROPEAN SEARCH REPORT

Application Number

EP 92 83 0612
Page 3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 5) |
| D,A | EP-A-0 412 058 (SIGMA-TAU)<br>6 February 1991<br>* the whole document *<br>--- | 1-13 | |
| D,A | CHEMICAL ABSTRACTS, vol. 111, 1989, Columbus, Ohio, US;<br>abstract no. 166767, 'Topological proline-based analogs of piracetam and their nootropic activities.'<br>* abstract *<br>& KHIM.-FARM. ZH.<br>vol. 23, no. 3, 1989,<br>pages 276 - 281<br>----- | 1-13 | |
| | | | TECHNICAL FIELDS SEARCHED (Int. Cl. 5) |

EPO FORM 1503 03.82 (P04E10)

EP 92 83 0612

-C-

INCOMPLETE SEARCH

Claims searched completely: 2-11

Claims searched incompletely: 1,12,13

LACK OF CONCISENESS

The definition of the following substituent(s) is too general and/or encompasses too broad a range of totally different chemical groups, only partly supported by examples given in the descriptive part of the application:

R, R1, R2

The number of theoretically conceivable compounds resulting from the combination of all claimed substituents of above list precludes a comprehensive search.
Guided by the spirit of application and the inventive concept as disclosed in the descriptive part of the present application the search has been limited to the following case(s):

R2 = H, formyl and benzyloxycarbonyl

R, R1 = as exemplified in the description and specified in claims 2-11

(Cf. Arts. 83,84 EPC, Rule 45 EPC, Guidelines Exam. Part B, Chapt. III, 3.6, 3.7)